# EUROPEAN PATENT APPLICATION

(11) **EP 2 255 817 A1**
(43) Date of publication of application: **01.12.2010**
(21) Application number: 09290321.0
(22) Date of filing: 05.05.2009
(51) Int. Cl.: A61K 31/727, C08B 37/10, C08B 37/00

(54) **Use of an acylated octasaccharide as antithrombotic agent**

(71) Applicant: Sanofi-Aventis, 75013 Paris (FR)
(72) Inventor: Mourier, Pierre, 75013 Paris (FR); Viskov, Christian, 75013 Paris (FR)
(74) Representative: Romanowski, Caroline

(57) **Abstract**

Summary

The instant invention relates to the use of the octasaccharide of formula (I), wherein Ac represents an acetyl group : 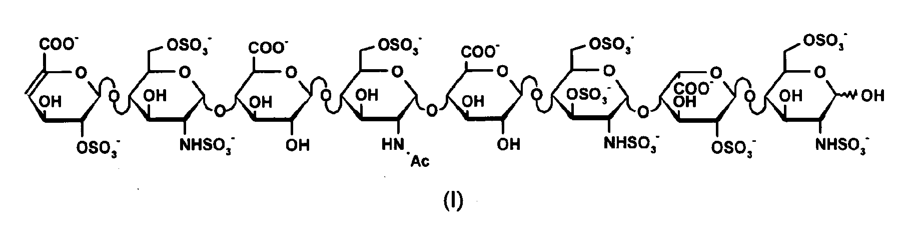 in its acid form or in the form of any one of its pharmaceutically acceptable salts, for the preparation of a medicament intended for the treatment and prevention of thromboses.

## Description

The instant invention relates to the use of an oligosaccharide, more specifically an acylated octasaccharide, as antithrombotic agent.

Clotting is a defense mechanism preventing excessive loss of blood and ingestion of microbes. Yet, inadvertent formation and dislocation of clots may be harmful; antithrombotic drugs prevent the formation and growth of clots.

Heparin and Low Molecular Weight Heparins (LMWHs) are the current standard therapy in the management of thromboembolic diseases. Their anticoagulant activity is exerted through inhibition of coagulation factors, mainly activated factor X (FXa) and thrombin (factor IIa). This inhibitory action is mediated by the specific interaction of heparin species with antithrombin (AT), a serine protease inhibitor of the serpin family.

These drugs derive from animal sources: unfractionated heparin (UFH) is isolated from tissues such as lungs or intestinal mucosa, from porcine or bovine origins. LMWHs, such as tinzaparin, ardeparin, dalteparin, enoxaparin, nadroparin or reviparin, are obtained by enzymatic or chemical depolymerisation of heparin.

Heparin and LMWHs are complex mixtures of molecules: they contain numerous sulfated polysaccharides, each of them being a polymer composed of a linear chain of monosaccharide residues. Therefore, the different polysaccharides present in heparin and in LMWHs vary in their lengths as well as in their chemical structures. The varying degree of sulfation and the presence of different 1→4 linked uronic acid and glucosamine disaccharide units give rise to a complex overall structure (J. Med. Chem., 2003, 46, 2551-2554).

Another class of antithrombotic drugs consists in synthetic oligosaccharides. Indeed, in the early 1980s it was determined that a unique pentasaccharide domain in some heparin chains is the minimal sequence required for binding and activating antithrombin III (Biochimie, 2003, 85, 83-89). Fondaparinux sodium is a synthetic analogue of this pentasaccharide, obtained through more than 60 steps of chemical synthesis. It is a selective inhibitor of factor Xa, commercialized for the prevention of thrombosis after orthopedic and abdominal surgery, for the prevention and treatment of deep vein thrombosis and pulmonary embolism, as well as for the treatment of coronary diseases.

Structure-based design has subsequently led to analogues with longer duration of action, such as idraparinux, displaying either selective factor Xa or dual Xa and IIa inhibition properties. The search for improved pharmacodynamic profiles lead to the synthesis of longer oligosaccharides, such as the clinical candidate SR123781 (hexadecasaccharidic compound), aiming at providing heparin mimetics that are more potent than heparin as regards antithrombin activity, but devoid of its side effects.

The Applicant has devised a novel approach for the identification of antithrombotic compounds. Starting from LMWHs, advances in analytical and separation methods have permitted the isolation and sequencing of some of their oligosaccharidic components, for which affinity for AT has been measured (M. Guerrini et al., J. Biol. Chem., 2008, vol. 283, No. 39, 26662-26675). Although its affinity for AT is known to be unrelated to the anticoagulant activity of a given oligosaccharide in plasma, the Applicant has surprisingly found that the oligosaccharide OCTA-4 disclosed by M. Guerrini *et a*/. (*ibid*.) is a compound with powerful anticoagulant properties, which is therefore particularly adapted for being used as an antithrombotic agent.

The invention therefore relates to the use of the oligosaccharide of formula (I) : 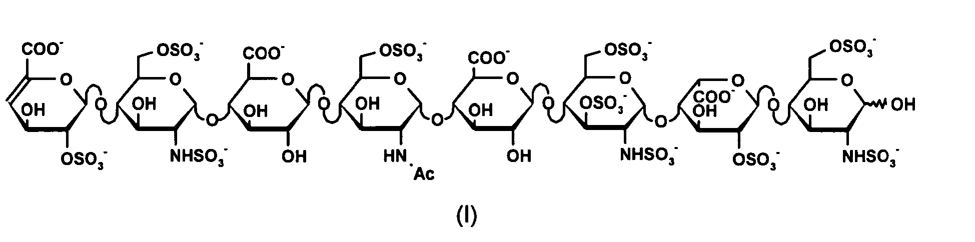 wherein Ac represents an acetyl group (i.e. a group of formula -COCH₃) and wherein the wavy line denotes a bond situated either below or above the plane of the pyranose ring, for the preparation of a medicament for use in the treatment and prevention of thromboses.

The oligosaccharide of formula (I) is an octasaccharide. The invention encompasses the use of the octasaccharide of formula (I) in its acid form or in the form of any one of its pharmaceutically acceptable salts. In the acid form, the carboxylate (-COO) and sulphate (-SO₃⁻) functional groups are respectively in the -COOH and -SO₃H forms.

The term "pharmaceutically acceptable salt" of the oligosaccharide of formula (I) is understood to mean an oligosaccharide in which one or more of the -COO- and/or -SO₃⁻ functional groups are bonded ionically to a pharmaceutically acceptable cation. The preferred salts for use in the instant invention are those for which the cation is chosen from the cations of alkali metals and more preferably still those for which the cation is sodium (Na⁺).

In accordance with the present invention, the compound of formula (I) can be obtained from a LMWH product by using orthogonal (combined) separation methods selected from Gel Permeation Chromatography (GPC), AT affinity chromatography and High Performance Liquid Chromatography (HPLC).

The following protocols describe in detail the preparation of the compound (I) according to the invention, in the form of a sodium salt, as described in J. Biol. Chem., 2008, vol. 283, No. 39, 26662-26675.

Octasaccharide of formula (I) is obtained by combining AT affinity chromatography and cetyltrimethylammonium-strong anion-exchange (CTA-SAX) chromatography on a semi-preparative scale, starting from octasaccharide gel permeation chromatography (GPC) fractions of enoxaparin. GPC of enoxaparin and the desalting conditions of the selected fractions were performed as described previously by Mourier, P.A.J. and Viskov, C. (Analytical Biochem., 2004, 332, 299-313). The octasaccharide fraction was chromatographed on an AT Sepharose column (40 x 5 cm) with a stepwise gradient of NaCl. The column was prepared by coupling human AT (1 g) to CNBr-activated Sepharose 4B (Sigma) according to Höök et al. (FEBS Letters, 1976, 66(1), 90-3). The low affinity portion was eluted from the column with a 0.25 M NaCl solution buffered at pH 7.4 with 1 mM Tris-HCl at 6 ml/min. The high affinity octasaccharide fractions were eluted with a step gradient of NaCl (range between 0.25 and 3 M NaCl, 1 mM Tris-HCl, pH 7.4). The NaCl gradient was monitored by conductivity measurements, and the octasaccharides in the effluents were detected by UV at 232 nm. Octasaccharides eluted above 145 mS/cm were used for the purification of the octasaccharide of formula (I). The final purification was achieved using CTA-SAX chromatography. CTA-SAX semi-preparative columns (25 x 5 cm or 25 x 2.2 cm) were prepared as described in Analytical Biochem. (*ibid*) and filled with Hypersil BDS C18 (5 µm particle size). Mobile phases for oligosaccharide separation were aqueous sodium methanesulfonate (Interchim) at concentrations varying between 0 and 2.5 M. The pH was adjusted to 2.5 by addition of diluted methanesulfonic acid. Separations were achieved at 40°C. Salt concentration in the mobile phase was increased linearly from 0 to 2.5 M over 60 min. Flow rate was 40 ml/min for 25 x 3-cm columns, and UV detection at 234 nm was used. Collected fractions were neutralized and desalted on Sephadex G-10 after a preliminary treatment on Mega Bondelut C18 cartridges (Varian).

The oligosaccharide used in the instant invention underwent pharmacological studies which demonstrated its antithrombotic properties and its value as therapeutically active substance.

### Anti-FXa activity in plasma :

The ability of the sodium salt of the oligosaccharide (I) .to accelerate AT-mediated FXa inhibition was analysed in nearly physiological conditions. The anti-FXa activity measurement was performed using the competitive chromogenic assay STA^{®}-Rotachrom^{®} Heparin (Diagnostica Stago Inc.) automated on a STA^{®}-R analyzer (Diagnostica Stago Inc.) according to the manufacturer's recommendation. Bovine FXa (Diagnostica Stago Inc.) was used. Fondaparinux was the reference material, obtained from commercial source marketed by GlaxoSmithKline. It was spiked at increasing concentrations (0.0218 - 0.0460 - 0.0872 - 0.1740 - 0.3490 - 0.4650 µmol/L) in normal pool human plasma (Hyphen). Dose response linearity was demonstrated. The oligosaccharide of the invention and fondaparinux were tested at 6 concentrations ranging from 0.0218 to 0.4650 µM. The concentration of AT in plasma milieu was 2.25 µM. The measured absolute anti-Xa activity of the purified oligosaccharide was expressed in IU/ml, according to European Pharmacopeia 6.0 (01/2008:0828). The relative anti-FXa activity was calculated from the ratio of the absolute activity *versus* that of fondaparinux.

In this test, the oligosaccharide used in the invention displays an absolute anti-FXa activity of 1.63 IU/ml. Its relative anti-Xa activity compared to fondaparinux is 2.07 fold.

The oligosaccharide of formula (I) according to the invention therefore displays high antithrombotic properties and is useful for the preparation of antithrombotic drugs. The resulting medicament is useful in particular in the treatment and prevention of thromboses, including venous thromboses (for example in the post-operative phase of surgical patients, in cancer patients or in medical patients with restricted mobility) and acute arterial thrombotic events, in particular in the case of myocardial infarction.

The oligosaccharide of formula (I) may be used alone or in combination with at least one other active principle selected from antithrombotic oligosaccharides, whether synthetic compounds (obtained by chemical, stepwise synthesis starting from appropriate mono- or oligosaccharidic building blocks) or compounds isolated from heparin or LMWHs sources.

The present invention, according to another of its aspects, also relates to a method for the treatment and prevention of the above pathologies, which comprises the administration to a patient of an effective dose of the oligosaccharide of formula (I) according to the invention, or a salt with a pharmaceutically acceptable salt thereof.

## Claims

1. Use of the oligosaccharide of formula (I): 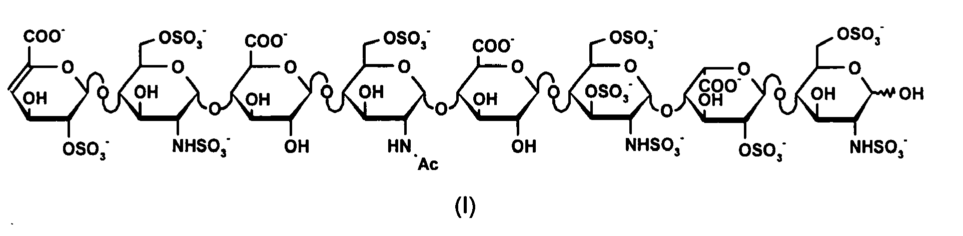 wherein Ac represents an acetyl group and wherein the wavy line denotes a bond situated either below or above the plane of the pyranose ring, said oligosaccharide being in its acid form or in the form of any one of its pharmaceutically acceptable salts, for the preparation of a medicament for use in the treatment and prevention of thromboses.

2. The use according to claim 1, wherein the oligosaccharide is in the form of its sodium salt.

3. The use according to claim 1 or claim 2, for the preparation of a medicament for use in the treatment and the prevention of venous thromboses and of acute thrombotic events.

4. The use according to any of claims 1 to 3, wherein the oligosaccharide of formula (I) is used in combination with at least another active principle selected from antithrombotic oligosaccharides.

5. A method for the treatment and prevention of thromboses, which comprises the administration to a patient of an effective dose of the oligosaccharide of formula (I) as defined in claim 1 or claim 2, or of an addition salt thereof with a pharmaceutically acceptable salt.
